(19) Europäisches Patentamt
European Patent Office
Office européen des brevets

(11) Publication number: 0 596 081 A1

(12) **EUROPEAN PATENT APPLICATION published in accordance with Art. 158(3) EPC**

(21) Application number: **93911512.7**

(51) Int. Cl.$^5$: **C12M 1/40**, C12Q 1/48

(22) Date of filing: **22.04.93**

(86) International application number: **PCT/ES93/00030**

(87) International publication number: **WO 93/22419 (11.11.93 93/27)**

(30) Priority: **23.04.92 ES 9200859**

(43) Date of publication of application: **11.05.94 Bulletin 94/19**

(84) Designated Contracting States: **AT BE CH DE DK FR GB GR IE IT LI LU NL PT SE**

(71) Applicant: **TABACALERA, S.A.**
**Alcala, 47**
**E-28014 Madrid(ES)**

(72) Inventor: **FERRERO CORRAL, José Maria**
**Alcalá, 47**
**E-28014 Madrid(ES)**
Inventor: **MONTOYA BAIDES, Angel**
**Alcalá, 47**
**E-28014 Madrid(ES)**

(74) Representative: **Carpintero Lopez, Francisco**
**HERRERO & ASOCIADOS, S.L.**
**Alcalá, 21**
**E-28014 Madrid (ES)**

(54) **ENZYMATIC BIOSENSOR FOR ASSAYING GLYCEROL IN LIOUID MEDIUM.**

(57) The disclosed biosensor is preferably applicable to determine quantitatively the presence of glycerol in a liquid medium, such as for example in the addition of the so-called "sauces" to tobacco during its facrication. Said product, "the sauce", contains among its components, wetting agents such as glycerol and propyleneglygol and, in this respect, it is very important to have means in order to control its concentration, including in the final product. The biosensor is comprised of a capsule wherein are held the active enzymes, and the capsule is provided with an agitator, th temperature of said capsule being controlled. The sample to be analyzed is introduced in the capsule in dosed amount. An amplifier is associated to an electrode, and the assembly is connected to a data processing system.

EP 0 596 081 A1

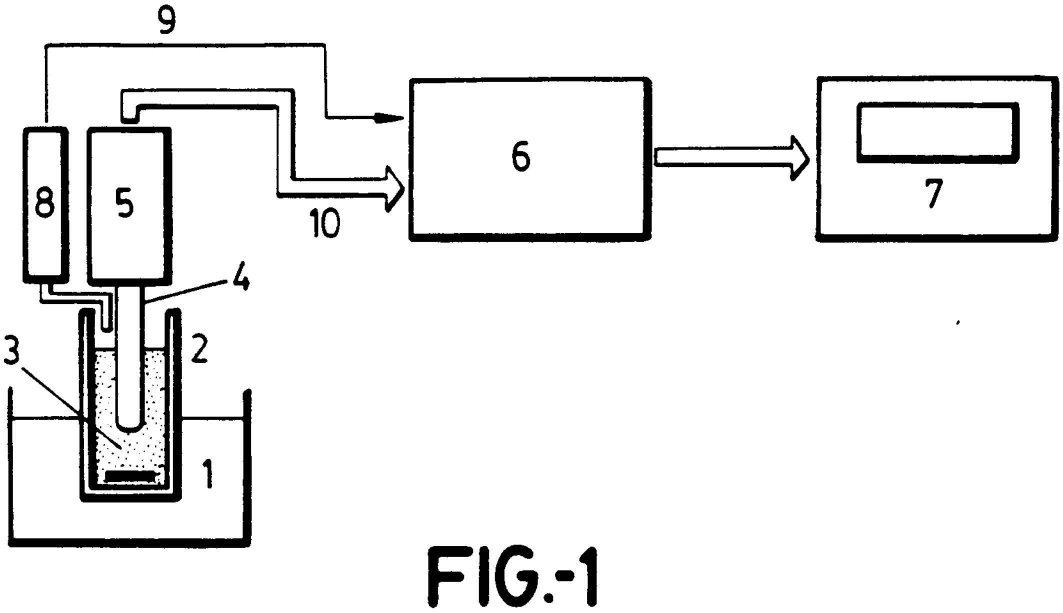


FIG.-1

## PURPOSE OF THE INVENTION

The invention refers to an enzymatic biosensor to determine the glycerol in a liquid medium and, in particular, a biosensor based on the enzymatic conversion of the glycerol by the sequential action of the glycerokinase which catalyses a first reaction whereby a first substrate, comprising the glycerol, is transformed into glycerol-3-phosphate, and the glycerol-3-phosphate oxidase which catalyses a second reaction whereby a second substrate, comprising the glycerol-3-phosphate, is transformed into dihydroxyacetone phosphate, consuming the oxygen in the medium, wherein said consumption is quantified by an ammetric electrode which generates an intensity signal indicating the oxygen concentration in the medium, and which is connected to the input of an electronic transresistance amplifier that applies an appropriate tension to the electrode according to the second enzymatic reaction and converting said intensity signal into a tension signal substantially proportional to said oxygen consumption. The output from said transresistance amplifier is connected to an analog-digital convertor which feeds codified signals to a data processing system operating with said signals, using a specifically developed program which handles the capture of data from said converter, using them to calculate the glycerol concentration in the reaction liquid medium by interpolating a set of curves obtained from previous trials on calibrated samples; in addition, said biosensor is characterized by the establishment of an initial oxygen level in said liquid medium by agitating it at a constant temperature which is controlled by a thermostatic system.

## ANTECEDENTS OF THE INVENTION

The quantitative calculation of the presence of glycerol in liquid media is essential to certain industrial processes where this substance is added to certain consumer articles. A typical case is the addition of so-called "dressings" to tobacco during its preparation which contain among other things humectants such as glycerol and propylene glycol; it is of the greatest importance to control their concentration, not just in such dressings but also in the end product. At present, this calculation is done with conventional analytic procedures, of which, because of its sensitivity, specificity and accuracy, gas chromatography stands out. Such procedures frequently make use of costly equipment operated by highly qualified personnel, and samples must be sent to a laboratory where they are prepared and analyzed with the resulting loss of time, since it is not economical or suitable to install such equipment on the production lines.

This invention overcomes this problem using a biosensor, understood as a device incorporating at least three basic components: a bioactive molecule able to recognize and react specifically with the substance to be analyzed (analyte), a physical-chemical transducer closely connected to the bioactive molecule and able to generate a signal when the specific interaction takes place with the analyte, and an electronic device which amplifies, upgrades and processes the signal generated, providing information on the concentration of analyte in the reaction medium.

As early as 1962, Clark and Lyon described an enzymatic sensor to determine urea, using the urease enzyme. In 1965, Kadish and Hall refined a system to measure glucose using glucose oxidase in a solution in which an oxygen electrode was submerged. In 1967, Updike and Hicks developed a system which introduced a major novelty, using the glucose oxidase in a polyacrylamide gel in close contact with the electrode.

Subsequently, although research along these lines continued, no significant novelties were provided either from the conceptual standpoint nor in terms of the real possibilities of application. However, at the beginning of the eighties, an enormous interest in biosensors arose, unquestionably boosted by the spectacular development of electronics and data processing. This made it possible to raise the real and immediate possibility of integrating the biological substrates into microcircuits to build bioelectronic sensors.

New transducer technologies (fibre-optics, acoustic wave surface crystals, selective field effect transistors, etc.), the development of new measuring techniques, and the spread of the use of novel biological systems (antibodies, bacteria, cellular organules, liposomes, etc.) have caused a major expansion of the areas of biosensor-related research.

Depending on the type of bioactive molecule used, biosensors can be enzymatic or immunological: the biosensor in this invention belongs to the former group.

Enzymatic reactions are relatively simple to detect and, in the right conditions, can generate signals which are reliable over short periods of time.

## A DESCRIPTION OF THE INVENTION

As mentioned in the introductory paragraph, the biosensor which is the subject of this invention uses two enzymes as bioactive molecules, which specifically catalyse two consecutive reactions, the latter of which consumes oxygen from the reaction medium.

These reactions can be shown schematically as follows:

$$\text{GLYCEROL} + \text{ATP} \xrightarrow{\text{GLYCEROKINASE}} \text{G-3-P} + \text{ADP}$$

$$\text{G-3-P} + O_2 \xrightarrow{\text{G-3-P-O}} \text{DHAP} + H_2O_2$$

where:

| | |
|---|---|
| ATP | : adenosine triphosphate |
| G-3-P | : glycerol-3-phosphate |
| G-3-P-O | : glycerol-3-phosphate-oxidase |
| DHAP | : dihydroxyacetone phosphate |

Because the initial oxygen concentration in the liquid reaction medium at rest is usually very low, a mechanical device, preferably of the magnetic type, is used to shake it, so as to increase said concentration by dissolution of the atmospheric oxygen. In addition, because the kinetics of the reactions and the activities of the enzymes depend on temperature and pH, the measuring process is done in thermostatic conditions and with the pH stabilized, by means of a suitable buffer. In the present invention, the working pH is set at 9, between those pH values at which enzymes are at maximum activity (8.5 for G-3-P-O and 10 for glycerokinase). It has been confirmed that the measurements are satisfactory in these conditions.

The transducer used is an electrode which is sensitive to partial oxygen pressure, and the signal generated is a current intensity which flows through the electrode when a constant potential appropriate to the oxidation-reduction reaction which takes place is applied to it. The electronic component is a transresistance amplifier circuit which simultaneously fulfils two functions; to generate said polarization potential and to measure the intensity by conversion to a proportional electrical tension. It is a feature that a high-performance integrated primary operational amplifier is used, which has an inverter input, a non-inverter input and an output: a constant negative tension is applied to said non-inverter input, obtained from a resistive tension divider connected between the ends of a reference Zener diode: the active terminal of the electrode is connected to a point which, in turn, is connected to said inverter input through an initial resistor and to the anode of the Zener diode by a second resistor, and said output through a circuit formed by a third resistor and a condenser in parallel, so that the electrode's active terminal is under a significantly constant negative tension of - 0.65 V and the current through it branches completely through said third resistor. At said output of the primary operational amplifier, there is a tension with a first continuous component which depends on the avalanche tension of the Zener diode and the values of the resistors in the circuit, and a second variable component which is equal to the product of the current intensity in the electrode through said third resistor, and where the combination of said third resistor and condenser in parallel constitutes a low-pass filter with a cutoff frequency of approximately 2 Hz. The circuit also includes a second integrated operational amplifier fitted in an inverse adder configuration, whose non-inverter input is connected to ground and its inverter input connected to a constant tension which can be adjusted, through a primary fixed weighting resistor, to the output of the primary operational amplifier through a second resistor, of variable weighting, and to its own output through a feedback resistor, so that the continuous level of the output signal from said second operational amplifier and the amplification factor of the output signal variable component of said primary operational amplifier can be adjusted separately.

Furthermore, the output tension of said second operational amplifier is digitally codified in an analog-digital convertor and the information obtained is captured by a data processing system which calculates the oxygen reduction and, therefore, the glycerol concentration in the sample being analyzed. The calculation is done by interpolation on a surface formed by a bunch of curves stored in the data processing system from trials on calibrated samples.

The authors have developed a number of prototypes of the biosensor, with which numerous glycerol measurements have been carried out on tobacco dressings, aromas and extracts. In order to validate the method, the results were compared systematically with those obtained from gas chromatography. The mean accuracy of the concentration values obtained from the biosensor were about 5% and the correlation with the reference chromatographic values was better than 0.99 with trial times of the order of two minutes.

## A DESCRIPTION OF THE DRAWINGS

The following is a description of the invention with reference to the accompanying drawings in which:

Figure 1 is a diagram showing the measuring system employed according to a preferential design of the biosensor which is the subject of this invention.

Figure 2 shows a circuit diagram of the transresistance amplifier used in such preferential design.

Figure 3 shows, at a scale of 1:1, the physical appearance of a prototype transresistance amplifier developed according to the preferential design of the invention.

Figure 4 shows a set of curves registered for different glycerol concentrations using the biosensor according to this invention.

## A PREFERENTIAL EMBODIMENT OF THE INVENTION

Figure 1 of the drawings shows in diagrammatic form the system of measurement used in a preferential embodiment of the invention, given by way of example and without limitation. A reaction capsule (2) which may consist of a test tube contains a buffer which is suitable for maintaining the activity of the enzymes used at the optimal level which, according to experiments performed by the authors of the invention, occurs at a Ph of 9. Said reaction capsule (2) is associated with a shaking device (3), preferably of the submergible magnetic type, in order to establish an initial oxygen concentration in the liquid reaction medium in said capsule due to dissolution of the atmospheric oxygen. The temperature of the reaction capsule (2) is controlled and stabilized by a device (19) which may consist of a thermostatic bath. The enzymes involved in the reactions can be added to the reaction capsule before calculating the glycerol, but should for preference be immobilized in the proximity of the measurement electrode (4) which, in this case, is an oxygen electrode. The sample for analysis is placed in the reaction capsule (2) using a dosing device (8) which may be an automatic injector, a calibrated pipette, or any other system which makes it possible, manually or automatically, to release measured quantities of sample into the reaction capsule (2). A specific feature of said dosing device (8) is that it must incorporate elements enabling a signal to be generated indicated the moment at which the sample is injected, so that the measuring process can begin.

The oxygen electrode (4) is connected to the input of a transresistance amplifier (5) which in turn is connected by a multiple wire cable (10) to a system for the capture and processing of data, and including at least an analog-digital convertor and a micro-processor or micro-controller able to handle the data captured, make the calculations necessary to determine the glycerol concentration of the sample by interpolation on a surface defined by curves obtained from calibrated samples, and to handle the presentation of results and auxiliary information through a display device (7). Clearly, blocks (6) and (7) can be unified in a computer with a data capture card. The line (9) connected between the dosing device (8) and the data capture and processing system (6) transmits a signal to indicate the moment when the sample is injected, in order to initiate the capture and measurement process, as already commented.

Figure 2 shows a diagram of the transresistance amplifier (10) used according to this invention, the components and connections of which have already been described. According to said diagram, the tension applicable to the non-inverter terminal of the initial AOP1 operational amplifier can be expressed as follows:

$$u_+ = -\frac{RB}{RA + RB} V_z \qquad (1)$$

since the tension at point M in relation to ground is that corresponding to the Zener diode D1 polarized in the inverse direction by the feed tension -V DC through resistor RO, and resistors RA and RB form a tension divider of coefficient RB/(RA + RB). In equation (I) $V_z$ is the avalanche tension of the Zener diode (preferably a reference Zener diode with a low temperature coefficient).

$$i_c = \frac{u_+ + V_z}{R2} + \frac{u_+ - u_3}{R3} \qquad (2)$$

where $u_3$ is the output tension of the AOP1 operational amplifier as shown in the figure.

Substitution of expression (1) in the previous equation gives the following equation for tension $u_3$:

$$u_3 = \frac{RAR3 - R2RB}{R2\,(RA + RB)} V_z - R3i_c \qquad (3)$$

showing that said output tension has two components, as follows: a continuous tension component which depends on the avalanche tension of the Zener diode D1 and on the values of the resistors in the first term of the second member, and a variable tension component equal to the product of the electrode current and feedback resistor R3.

In order to reject unwanted high frequency signals, condenser C is incorporated in parallel with resistor R3 so that the circuit transfer function is assigned to a low-pass filter with a cut-off frequency equal to 1/2ΠR3 C which, with the components selected, is approximately 2 Hz.

The output of the first operational amplifier AOP1 is connected to the inverter input of a second operational amplifier AOP2 through an adjustable resistor R6. A tension is also applied to said inverter input which is adjustable between a symmetrical positive and negative value, through a resistor R7. The inverter input of said second operational amplifier AOP2 is connected to ground and its output is connected through a resistor R8 to the inverter input. The configuration described corresponds to an inverter adder circle with the following transference function:

$$u_o = -\frac{R8}{R6} u_3 + \frac{R8}{R7} u_{DC} \qquad (4)$$

where $u_0$ is the output tension of operational amplifier AOP2 and $u_{DC}$ is the adjustable continuous tension supplied by the combination of resistors R4 and R5, the Zener diodes D2 and D3, and the potentiometer Rp, and said continuous tension appears at the cursor of said potentiometer.

This configuration permits independent adjustment of the level of continuous tension of the signal $u_0$ of the output from the circuit described, and the amplification factor R8/R6 which is applied to the variable component in order to adapt the signal to the requirements of the analog-digital convertor to whose input the transresistance amplifier output is connected.

Figure 3 shows, at real scale, the physical appearance of a prototype transresistance amplifier developed by the invention's authors. The electronic components are arranged on a printed circuit board in a small metal housing, and the adjustments in the continuous level and amplification or gain can be observed. The electrode is connected to the amplifier through a coaxial connector, as shown on the left of the figure. The feed and amplifier output tensions correspond to the terminals of a multiple connector seen on the right of the figure which is connected to the data capture and treatment system (multiple line 10, figure 1).

Figure 4 reproduces several curves for the transresistance amplifier output signal according to the time for various glycerol concentrations, presented by the data capture and processing system which, in the prototype developed, consisted of a computer with a standardized data capture card. The programs specifically developed by the authors enable storage of multiple curves of this type, obtained from trials with calibrated samples, interpolating unknown samples using a three-dimensional interpolation algorithm on

the surface defined by the standardized set of curves (this is in fact a surface, since the function of oxygen concentration in the reaction medium depends on two independent variables - time and glycerol concentration). As already mentioned, the capture and calculation process is initiated when the data processing system receives a signal from the sample injector dosing device (8).

Although the invention has been explained with reference to a particular design for application in the calculation of glycerine in tobacco dressings, aromas and extracts, it is understood as being applicable to calculating the glycerol in any liquid medium. The components and circuits described may be modified or substituted by others which perform the same function, without thereby affecting the content and the scope of application of the invention, as defined in the attached claims.

## Claims

1. An enzymatic biosensor for measuring the glycerol in liquid media and, in particular, a biosensor based on the enzymatic transformation of the glycerol by the sequential action of the glycerokinase which catalyses an initial reaction in which a first substrate, comprising the glycerol, is transformed into glycerol-3-phosphate, and the glycerol-3-phosphate oxidase which catalyses a second reaction whereby a second substrate, comprising the glycerol-3-phosphate, is transformed into dihydroxyacetone phosphate, consuming the oxygen in the medium, wherein said consumption is quantified by an ammetric electrode which generates an intensity signal indicating the oxygen concentration in the medium, and which is connected to the input of an electronic transresistance amplifier that applies an appropriate tension to the electrode according to the second enzymatic reaction and converting said intensity signal into a tension signal substantially proportional to said oxygen consumption. The output from said transresistance amplifier is connected to an analog-digital convertor which feeds codified signals to a data processing system operating with said signals, using a specifically developed program which handles the capture of data from said converter, using them to calculate the glycerol concentration in the reaction liquid medium by interpolating a set of curves obtained from previous trials on calibrated samples.

2. An enzymatic biosensor for measuring the glycerol in liquid media as set forth in claim 1, wherein an initial oxygen level is established in said liquid medium by agitating it at a constant temperature which is controlled by a thermostatic system, and because the enzymatic reactions take place in a medium buffered at a pH of approximately 9.

3. An enzymatic biosensor for measuring the glycerol in liquid media as set forth in claim 1 wherein, as well, a circuit is employed as transresistance amplifier which incorporates a high-performance integrated primary operational amplifier with an inverter input, a non-inverter input and an output: a constant negative tension is applied to said non-inverter input, obtained from a resistive tension divider connected between the ends of a reference Zener diode: the active terminal of the electrode is connected to a point which, in turn, is connected to said inverter input through an initial resistor and to the anode of the Zener diode by a second resistor, and said output through a circuit formed by a third resistor and a condenser in parallel, so that the electrode's active terminal is under a significantly constant negative tension of -0.65 V and the current through it branches completely through said third resistor. At said output of the primary operational amplifier, there is a tension with a first continuous component which depends on the avalanche tension of the Zener diode and the values of the resistors in the circuit, and a second variable component which is equal to the product of the current intensity in the electrode through said third resistor, and where the combination of said third resistor and condenser in parallel constitutes a low-pass filter with a cutoff frequency of approximately 2 Hz.

4. An enzymatic biosensor for measuring the glycerol in liquid media as set forth in claim 2, wherein the circuit also includes a second integrated operational amplifier fitted in an inverter adder configuration, whose non-inverter input is connected to ground and its inverter input connected to a constant tension which can be adjusted, through a primary fixed-weighting resistor, to the output of the primary operational amplifier through a second, variable-weighting resistor, and to its own output through a feedback resistor, so that the continuous level of the output signal from said second operational amplifier and the amplification factor of the output signal variable component of said primary operational amplifier can be adjusted separately.

5. An enzymatic biosensor for measuring the glycerol in liquid media as set forth in claim 1, wherein said data processing system is a micro-processor.

6. An enzymatic biosensor for measuring the glycerol in liquid media as set forth in claim 1, wherein said data processing system is a micro-processor.

7. An enzymatic biosensor for measuring the glycerol in liquid media as set forth in claim 1, wherein said data processing system is a computer.

8. An enzymatic biosensor for measuring the glycerol in liquid media as set forth in any of the previous claims, wherein the liquid medium is a tobacco dressing, aroma or extract.

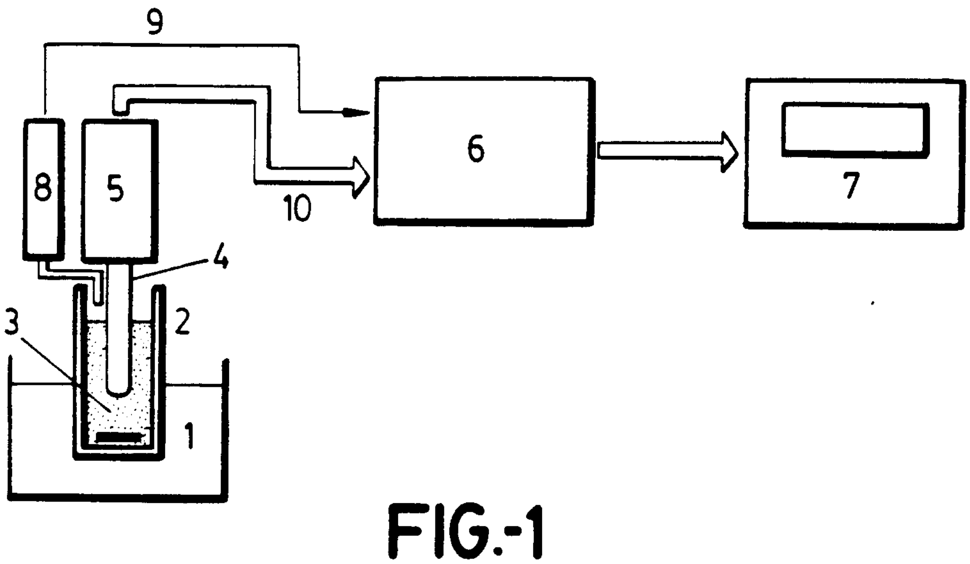
9
8
5
4
3
2
1
10
6
7

FIG.-1

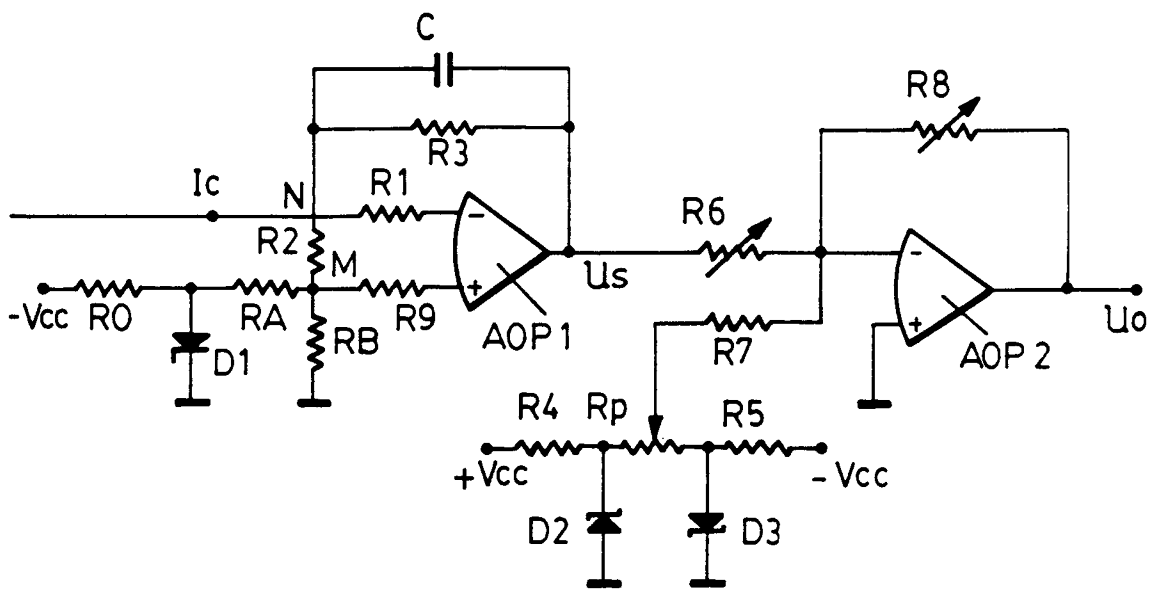
C
R3
R8
Ic
N
R1
R2
M
R6
-Vcc
R0
RA
R9
Us
D1
RB
AOP 1
R7
AOP 2
Uo
R4
Rp
R5
+Vcc
-Vcc
D2
D3

FIG.-2

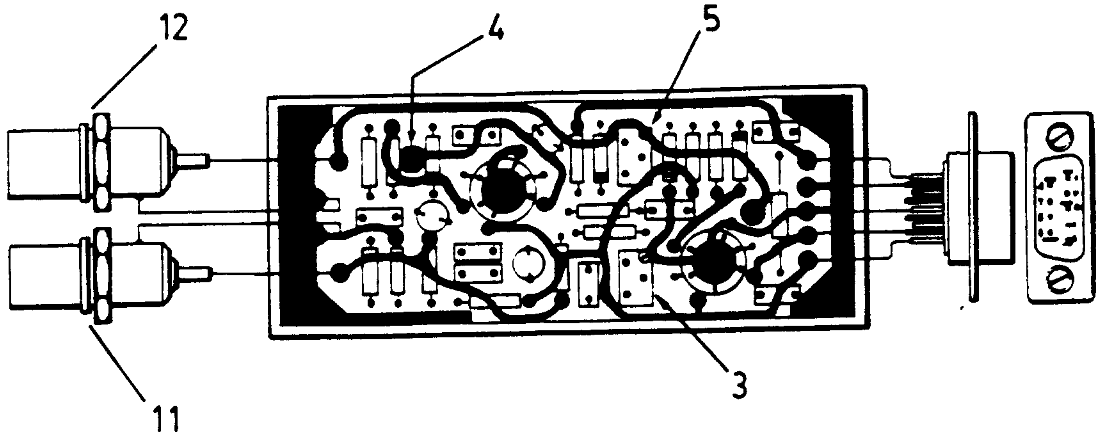
12
4
5
11
3

FIG.-3

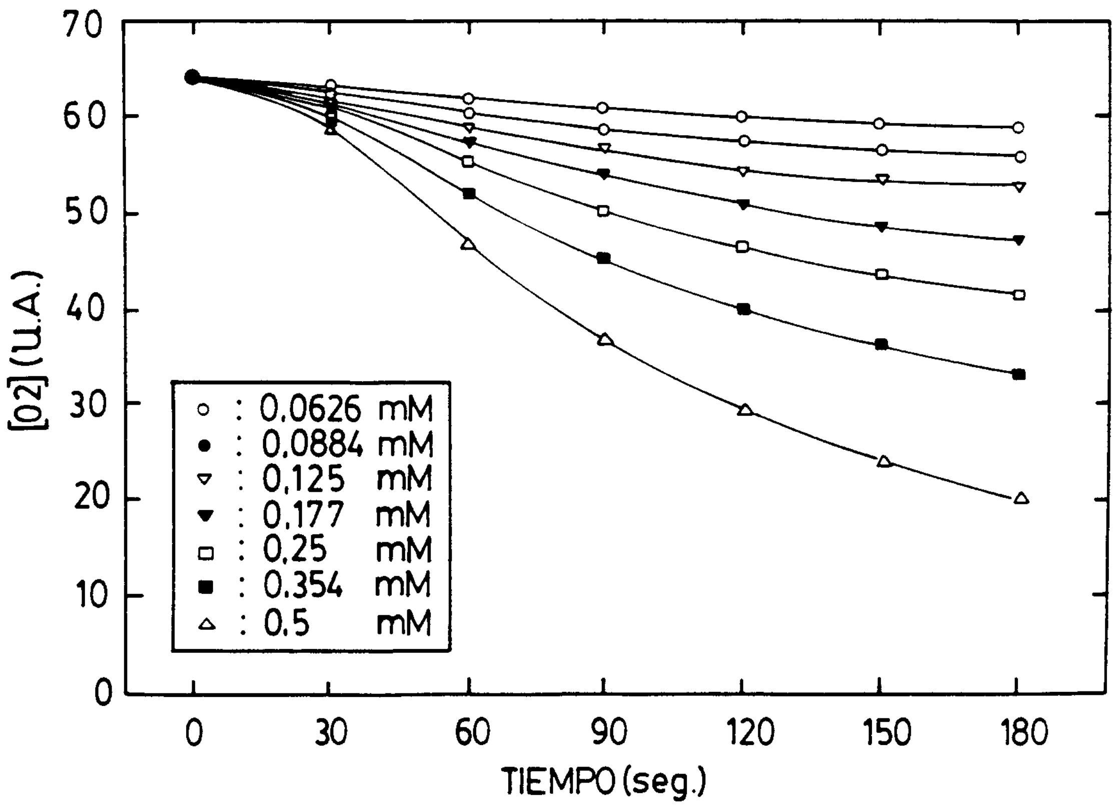
70
60
50
40
30
20
10
0
[02] (U.A.)
○ : 0,0626 mM
● : 0,0884 mM
▽ : 0,125 mM
▼ : 0,177 mM
□ : 0,25 mM
■ : 0,354 mM
△ : 0,5 mM
0
30
60
90
120
150
180
TIEMPO (seg.)

FIG.-4

**INTERNATIONAL SEARCH REPORT**

International application No.
PCT/ES 93/00030

**A. CLASSIFICATION OF SUBJECT MATTER**

Int. Cl.$^5$ C12M1/40; C12Q1/48

According to International Patent Classification (IPC) or to both national classification and IPC

**B. FIELDS SEARCHED**

Minimum documentation searched (classification system followed by classification symbols)

Int. Cl.$^5$ C12Q

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | DAROLD WOBSCHALL 'CIRCUIT DESIGN FOR ELECTRONIC INSTRUMENTATION' 1987 , MC GRAW-HILL BOOK COMPANY , NEW YORK see page 149 - page 150 | |
| A | PATENT ABSTRACT OF JAPAN vol. 8, No. 248 (P-313)14 November 1984 & JP,A,59 120 947 ( SHARP KK ) 12 July 1984 see abstract | |
| A | EP,A,0 035 480 (SVEN-OLOF ENFORS) 9 September 1981 | |
| P,A | JOURNAL OF ELECTROANALYTICAL CHEMISTRY vol. 344, 15 January 1993, LAUSANNE pages 161 - 166 W. J. ALBERY ET AL. 'A DIALYSIS ELECTRODE FOR GLYCEROL' | |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September 1993 (14.09.93) | 22 September 1993 (22.09.93) |
| Name and mailing address of the ISA/ EUROPEAN PATENT OFFICE Facsimile No. | Authorized officer Telephone No. |